# EUROPEAN PATENT SPECIFICATION

(11) **EP 1 658 091 B1**
(45) Date of publication and mention of the grant of the patent: **27.10.2010**
(21) Application number: 04743254.7
(22) Date of filing: 05.07.2004
(51) Int. Cl.: A61K 38/10, A61K 38/17, A61P 37/02

(54) **PHARMACEUTICAL COMPOSITIONS COMPRISING AN EPITOPE OF PLATELET GPIIIA PROTEIN**
EIN EPITOP DES BLUTPLÄTTCHENPROTEINS GPIIIA UMFASSENDE PHARMAZEUTISCHE ZUSAMMENSETZUNGEN
COMPOSITIONS PHARMACEUTIQUES CONTENANT UN EPITOPE DE LA PROTEINE GPIIIA DE PLAQUETTE SANGUINE

(30) Priority: 04.07.2003 GB 0315754
(43) Date of publication of application: 24.05.2006
(73) Proprietor: ABERDEEN UNIVERSITY, Aberdeen AB24 3FX (GB); THE COMMON SERVICES AGENCY FOR THE SCOTTISH HEALTH SERVICE, Edinburgh Lothian EH12 9EB (GB)
(72) Inventor: URBANIAK, Stanislaw, Joseph, Aberdeen AB11 7SE (GB); BARKER, Robert, Norman, Inverurie AB51 8TD (GB); SUKATI, Hosea Dep. of Medicine and Therapeutics,, Foresterhill, Aberdeen AB9 2ZW (GB)
(74) Representative: Peebles, Katrina
(86) International application number: PCT/GB2004/002909
(87) International publication number: WO 2005/002613

(56) References cited:
- WO-A-90/00178
- WO-A-90/12593
- WO-A-93/12127
- WATKINS N.A. ET AL.: "HPA-1a phenotype-genotype discrepancy reveals a naturally occurring Arg93Gln substitution in the platelet beta-3 integrin that disrupts HPA-1a epitope." BLOOD, vol. 99, no. 5, 1 March 2002 (2002-03-01), pages 1833-1839, XP002304950

## Description

The present invention relates to compositions and methods for the treatment of diseases or illness relating to platelets, in particular, fetomaternal alloimmune thrombocytopenia.

Platelets play an important part in primary and secondary haemostatis. Platelet blood group antigens are present on the surface glycoproteins and are expressed as pairs of alleles [a or b], which are co-dominant. This results in individuals having human platelet antigen (HPA) genotypes a/a, a/b or b/b. Individuals that are negative for a particular allele, may be immunised if exposed to the antithetical allele (by pregnancy or blood transfusion) and produce alloantibody responses. These antibodies are clinically important and may cause fetomaternal alloimmune thrombocytopenia (FMAIT) , or reactions and resistance to platelet transfusions given to prevent bleeding.

The HPA-1a antigen has been recognized as the most immunogenic and clinically important antigen of the HPA system in Caucasians. Localised on platelet membrane GPIIIa, this antigen frequently causes FMAIT when incompatibility between fetal and maternal platelet antigens occurs. HPA-1a, regarded as a conformational B-cell antigen, arises as a result of polymorphism, causing a change from cytosine to thymine at position 196 of the gene for platelet glycoprotein IIIa. This causes the substitution of proline (HPA-1b) for leucine (HPA-1a) at amino acid 33 of the GPIIIa protein. FMAIT occurs when the antiplatelet antibodies of a sensitised HPA-1b1b mother cross the placenta and cause thrombocytopenia in an HPA-1a fetus, or in the neonate.

Approximately 2-3% of the population are HPA-1b homozygous and 10% develop anti-HPA-1a antibodies during pregnancy. The production of the alloantibody is strongly associated with inheritance of MHC genes, with greater than 99% carrying DRB3*0101, suggesting that the development of the alloantibody is HLA restricted. Previous work has been limited to study of the peptide-MHC Class II. It has been shown, using recombinant DRB3*0101 molecules and peptides corresponding to the HPA-1a and HPA-1b polymorphism, that the MHC class II-molecule binds only to peptide containing the HPA-1a polymorphism. It has been shown that only 35% of HPA-1b homozygous women carrying DRB3*0101 will actually develop alloantibodies indicating that the MHC class II presentation of peptides to T-cells is complex. In particular, such studies of MHC class II binding have not accounted for the apparent protective effect of DRB1*15 alleles.

The estimated incidence of severe alloimmune thrombocytopenia is approximately 1 in 1100 neonates. The clinical manifestations range from mild purpura to intracranial haemorrhage, which in severe cases ends up in death or lifelong disability. In FMAIT the first-born is affected in 40-60% of cases. At present there are no preventative measures or antenatal routine screening procedures in place to identify women at risk of HPA-1a alloimmunisation. The diagnosis of affected babies is made after the unexpected discovery of severe thrombocytopenia after the baby has been born. Transfusing an appropriate dose of ABO, Rh compatible and HPA-la negative platelets is usually the treatment of choice after the baby is born, which can be distressing to both baby and mother. Intrauterine platelet transfusion and administration of high dose intravenous immunoglobulin (IVIg) and steroids to mothers is controversial and is generally reserved for cases in which the estimated risk of severe fetal and neonatal thrombocytopenia.is considerable. Presently, there are no reliable predictors of severe fetal disease and antenatal treatment is based on measurement of maternal antibody levels and fetal platelet counts which all have their limitations.

Considerable attention has been directed to the characterisation and definition of the GPIIIa molecule and to resolve precisely the molecular structure of the alloantigenic B-cell determinants on GPIIIa responsible for eliciting anti HPA-1a antibodies. While the molecular identification of the alloantigenic epitope for anti-HPA-1a alloantibodies and the role of B-cells have improved understanding of the structural requirements for recognition by pathogenic alloantibodies, studies to understand T-cell involvement are few and inconclusive.

It is an object of the present invention to overcome the disadvantages of the prior art.

According to an aspect of the present invention there is provided use of an immunologically effective human platelet antigen (HPA) or a peptide fragment thereof in the manufacture of a medicament for the prevention or management of a condition caused by exposure to an antithetical allele of a platelet by transfusion or during pregnancy by tolerisation wherein the medicament is formulated for delivery through non-invasive routes.

The present invention allows the prevention or management of a condition caused by exposure to an antithetical allele (e. g. FMAIT or platelet transfusion refractoriness) by tolerisation. Tolerisation is a non-invasive method, which involves providing relatively small amounts of a peptide or protein to a patient generally through the mucosal tissue. The patient's immune system then over a period of time becomes tolerant to the peptide or protein and, therefore, does not consider the protein or peptide foreign. Accordingly, no effector immune response is raised.

If, for example, a women is HPA-1b1b then immunologically effective fragments of the HPA-1a antigen can be administered to her by a tolerogenic route (e.g. mucosa). If the woman then becomes pregnant with a fetus which is HPAlalb then the fetus is unlikely to develop FMAIT because the woman is less likely to mount an immune response against the fetus's platelets. Further, if a women had already had a baby with FMAIT then she could be administered an immunologically effective fragment or fragments of the HPA-1a antigen in order to reduce the likelihood of a subsequent baby being born with FMAIT.

Conditions which compositions of the present invention may prevent or manage, include FMAIT,post-transfusion purpura due to anti-HPA-1a, or platelet refractoriness.

Tolerisation as a preventative or management therapy for these conditions is more acceptable and less distressing than having to give a matched transfusion, steroid treatment or IVIgG, which are the present treatments for FMAIT.

The treatment for platelet refractoriness (resistance) involves extensive platelet typing and administration of matched platelets, which is time consuming and expensive.

Post-transfusion purpura is potentially life-threatening and the current treatment of choice would be the avoidance of platelet transfusions, and administration of IVIgG.

FMAIT is a disease caused by platelet destruction as a result of the mother mounting an immune response to the neonate's platelets.

HPA is a class of platelet proteins that changes with the genotype.. This can result in alloantibodies being produced if an individual who is negative for a particular HPA allele is exposed to that allele. In this connection, only an individual who is homozygous (i.e. 1a1a or 1b1b) is at risk of alloimmunisation.

The HPA, which is to be used in the composition of the present invention, may be but is not limited to HPA-1a, HPA-1b HPA-2a, HPA-2b, HPA-3a, HPA-3b, HPA-4a, HPA-4b, HPA-5a, HPA-5b, HPA-6a, HPA-6b, HPA-7a, HPA-7b, HPA-8a, HPA-8b, HPA-9a, HPA-9b, HPA-10a, HPA-10b, HPA-11a, HPA-11b or their derivatives, or sequences cross-reactive with them.

The "a" genotype is the most common genotype of the two.

HPA-1a is found predominantly in Caucasians and has the single nucleotide polymorphism cytosine to thymine at position 196 that results in a substitution of proline (HPA-1b) for leucine (HPA-1a) at position 33.

HPA-4a is found predominantly in Orientals and has the single nucleotide polymorphism guanine to adenine at position 526 that results in a substitution of arginine (HPA-4a) for glutamine (HPA-4b) at position 143.

HPA-2 has the single nucleotide polymorphism cytosine to thymine at position 524 that results in a substitution of threonine (HPA-2a) for methionine (HPA-2b) at position 145.

HPA-3 has the single nucleotide polymorphism thymine to guanine at position 2622 that results in a substitution of isoleucine (HPA-3a) for serine (HPA-3b) at position 843.

HPA-5 has the single nucleotide polymorphism guanine to adenine at position 1648 that results in a substitution of glutamic acid (HPA-5a) for lysine (HPA-5b) at position 505.

HPA-6 has the single nucleotide polymorphism guanine to adenine at position 1564 that results in a substitution of arginine (HPA-6a) for glutamine (HPA-6b) at position 489.

HPA-7 has the single nucleotide polymorphism cytosine to guanine at position 1267 that results in a substitution of proline (HPA-7a) for alanine (HPA-7b) at position 407.

HPA-8 has the single nucleotide polymorphism thymine to cytosine at position 2004 that results in a substitution of arginine (HPA-8a) for cysteine (HPA-8b) at position 636.

HPA-9 has the single nucleotide polymorphism guanine to adenine at position 2603 that results in a substitution of valine (HPA-9a) for methionine (HPA-7b) at position 837.

HPA-10 has the single nucleotide polymorphism guanine to adenine at position 281 that results in a substitution of arginine (HPA-7a) for glutamine (HPA-7b) at position 62.

HPA-11 has the single nucleotide polymorphism guanine to adenine at position 1996 that results in a substitution of arginine (HPA-11a for histidine (HPA-11b) at position 62.

Conveniently the HPA has a genotype HPA-1a.

Helper T-cells recognise antigen as linear peptides bound to MHC molecules and, immunodominant T-cell epitopes on an antigen can be identified, by challenging helper T-cells *in* vitro with linear peptides derived from the protein sequence of the antigen. The protein sequence of GPIIIa, which is the carrier molecule for HPA-1a, has already been determined, enabling the preparation of synthetic HPA-1a GPIIIa peptides corresponding to the HPA-1a polymorphic region of the GPIIIa molecule.

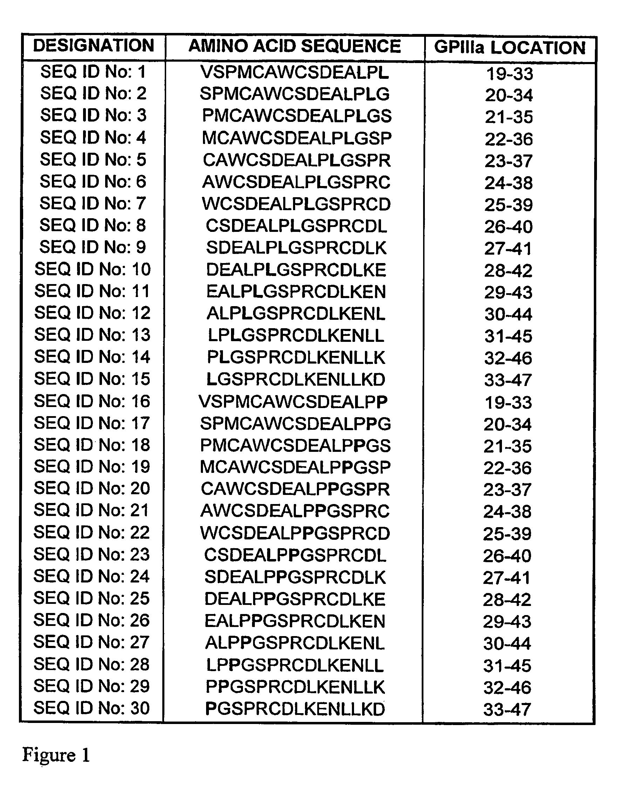

In the present invention a set of linear peptides with the polymorphism at every possible position in 15 mer peptides (see Figure 1) was derived, and this was successful in identifying Leu-33-peptide specific responses. These 15 mer peptides have been used to map alloreactive helper T-cell epitopes on the platelet membrane GPIIIa polymorphic region. Synthetic peptides containing these epitopes can be used to prepare a tolerogenic composition to prevent or reverse the alloantibody response to HPA-1a in susceptible individuals in vivo.

Conveniently, the HPA has sequence SEQ ID No:1, 2, 3, 4, 5, 6 or 7.

Peptides with these sequences were found to be particularly efficient at stimulating proliferation of Peripheral Blood Mononuclear Cells (PBMCs), which indicates that they would be useful in the tolerisation of a subject against a protein containing these peptides or the peptides themselves. T-cells recognise peptides containing the polymorphism when the polymorphism is in the C-terminal part of the peptide.

Conveniently, the HPA or fragment thereof is disposed in a pharmaceutically acceptable vehicle.

Conveniently, the vehicle is selected such that the composition is in a form which can be delivered through mucosal tissue. This may involve being administered nasally, orally, rectally, vaginally, topically or by a spray.

Mammals may be tolerised to certain proteins or peptides by uptake of relatively small amounts of the specific protein or peptide through, for example, mucosal tissue or via the gut. Accordingly, proteins or fragment thereof for use in the present invention can be administered to a subject in need of tolerisation via, for example, mucosal tissue and effectively tolerise the subject without causing an effector immune response.

The present invention will now be described, by way of illustration only, with reference to the following examples and the accompanying figures.
Figure 1 shows the panel of 15-mer peptides used in Example 1 of the present invention. The sequence was taken from Frachet et al, Mol.Bio. Rep. 1990, vol 14 27-33 (accession no. M35999).
Figure 2 shows a summary of HPA-1a and 1b peptides eliciting PBMC proliferation from HPA-1b1b women.
Figure 3 shows the percentage of HPA-1a alloimmunised HPA-1b1b women exhibiting reactivity to HPA-1a peptides compared to HPA-1b peptides.
Figure 4 shows a summary of HPA-1a and 1b peptides eliciting PBMC proliferation from control subjects.
Figure 5 shows the proliferative responses of PBMC from 3 HPA-1a alloimmunised HPA-1b1b women to HPA-1a/1b peptides.
Figure 6 shows the proliferative responses of PBMC from 3 HPA-1a1b unimmunised Control Donors to HPA-1a/1b peptides.
Figure 7 shows the proliferative responses of PBMC from HPA-1a alloimmunised HPA-1b1b women to HPA-1a/1b peptides compared to Control Donors.
Figure 8 shows the flowcytometric determination of phenotype of proliferated lymphocytes from patient NAIT-T40 when stimulated with SEQ ID No:1 and 2.

### Example 1

### Patients and Control Subjects

Peripheral blood mononuclear cells (PBMC) were isolated from samples of whole blood obtained from 21 HPA-1b1b patients, 14 of whom had developed anti-HPA-1a alloantibody during pregnancy, the remaining 7 of whom were found to be antibody negative. The cells were stimulated with a panel of 15-mer peptides corresponding to the sequences of the HPA-1a and 1b alloantigens containing Leu33 and Pro33 respectively (see Figure 1). Two categories of control donors were utilised; 1 HPA-1b1b unimmunised male donor and 11 HPA-1a randomly selected female donors. Testing of both patients and controls was carried out in cell culture conditions designed to favour proliferation by previously activated T-cells, rather than primary responses.

### Assay of anti-HPA-1a alloantibodies

Anti-HPA-1a alloantibody concentration in sera was measured by an enzyme-linked immunosorbent assay (ELISA) using maxisorp microtitre plates coated with 0.8µg/ml purified GPIIb/IIIa. 100 µL of diluted serum was added to duplicate coated and uncoated wells. Positive and negative antibody controls were included. Commercially available ELISA reagents were obtained from Axis-Shield Dundee, Scotland and the assay carried out as described by Bessos et al in Thrombosis Research, 1990, vol 59, 497-507.

### HPA Genotyping

Alleles encoding HPA-1 were determined using a PCR genotyping protocol as designed by Meyer et al in Transfusion,1999, vol 39, 1256-1258. PCR amplification reactions were carried out in a final volume of 30 µL.

### Preparation of Antigens and Mitogens for Peripheral Blood Mononuclear Cell (PBMC) Stimulation

The human platelet membrane GPIIIa amino acid sequence reported by Frachet et al Mol. Biol. Rep. 1990, vol 14, 27-33 (genebank accession no: M35999) was used as the basis for production of the HPA-1a and HPA-1b GPIIIa synthetic peptides. Two panels of 15-mer peptides were synthesised corresponding to the sequences of the HPA-1 polymorphic region, with either the Leu-33 or Pro-33 at each possible position (see Figure 1). One set of the 15-mer peptides contained the leucine polymorphism and the other set of 15 contained the proline polymorphism. To ensure purity each panel was synthesized by f-moc chemistry. The peptides were reconstituted to a concentration of 2.0 mg/ml and used for stimulation of PBMC at 20 µg/mL in culture.

Control antigens purified protein derivative (PPD) (Statens Serumintitut, Denmark) and keyhole limpet hemocyanin (KLH) (Calbiochem Behring La Jolla, CA) were obtained commercially, dialysed against phosphate buffered saline (PBS) pH 7.4, and filter sterilised before addition to cultures at 20µg/mL as recall and primary response antigens respectively. Concanavalin A (Con A) (Sigma, Poole, Dorset, UK), a T-cell mitogen, was used to stimulate PBMC at a concentration of 20µg/mL in culture as a positive control response.

### Isolation of Peripheral Blood Mononuclear Cells

Mononuclear cells were recovered from samples of peripheral blood of all patients and control donors by density gradient centrifugation technique (Lymphoprep; Nycomed, Denmark) according to the manufacturer's instructions. The cells were then manually counted by hemocytometer. Cell viability was determined by trypan blue exclusion; in all counts it was found to be approximately 90 to 100%.

### T-cell Proliferation Assay

Peripheral blood mononuclear cells were cultured at a concentration of 1.25 x10⁶ cells per mL in Alpha Modification of Eagle's Medium (Sigma Diagnostic, St. Louis, MO, USA.) supplemented with 4 mmol/l L-glutamine (Gibco, Paisley, UK), 100µg/ml streptomycin sulphate (Sigma Diagnostic, St. Louis, MO, USA) , 100u/mL sodium benzylpenicillin G (Sigma Diagnostic, St. Louis, MO, USA), 20mmol/L HEPES, PH 7.2 (Sigma Diagnostic, St. Louis, MO, USA) and 5% autologous serum or AB serum. Twelve microlitres of synthetic HPA-1a/1b peptides and control antigens (PPD, KLH and ConA) were each added to appropriate wells of the microtitre plates before the cell culture suspension was layered. The culture plates were incubated at 37°C in a humidified atmosphere of 95% air and 5% CO₂ for 5 days. T-cell proliferation in cultures was estimated from the incorporation of ³H-thymidine in triplicate 100µL samples withdrawn from the plates 5 days after stimulation. T-cell proliferation results were presented either as the mean counts per minute (CPM) ± SD of the triplicate samples or as a stimulation index (SI) expressing the ratio of mean CPM in stimulated versus unstimulated control cultures. A SI >3 was interpreted as representing a positive response.

### Characterization of Proliferating Lymphocytes by Flow Cytometry

Culture conditions for activation of PBMC from the patients were created by stimulating PBMC with 20µg/ml synthetic HPA-1a and HPA-1b peptides in 1ml wells on a 48-well flat bottomed plate at a concentration of 1.25 x 10⁶ cell/ml in 1ml aliquots. Unstimulated PBMC, or PBMC incubated with respective peptides, were examined for CD4 surface expression and the expression of CD71 activation markers by 3 colour flow cytometry. Thresholds for positives were set using isotype matched control antibodies. Immunophenotyping of 10,000 cells was analysed for the lymphocyte markers CD3 and the surface expression of CD4 and the activation marker CD71.

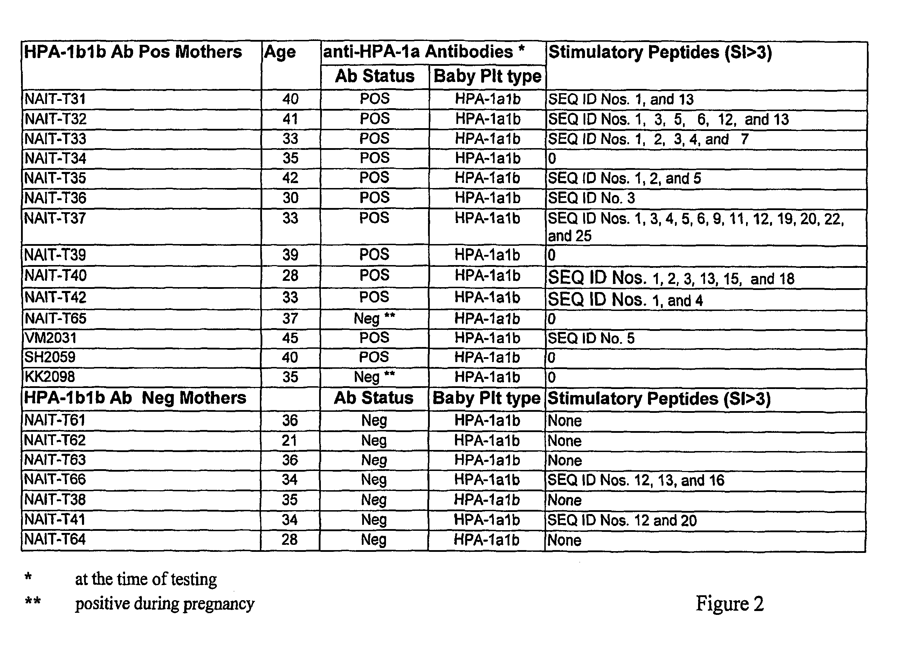

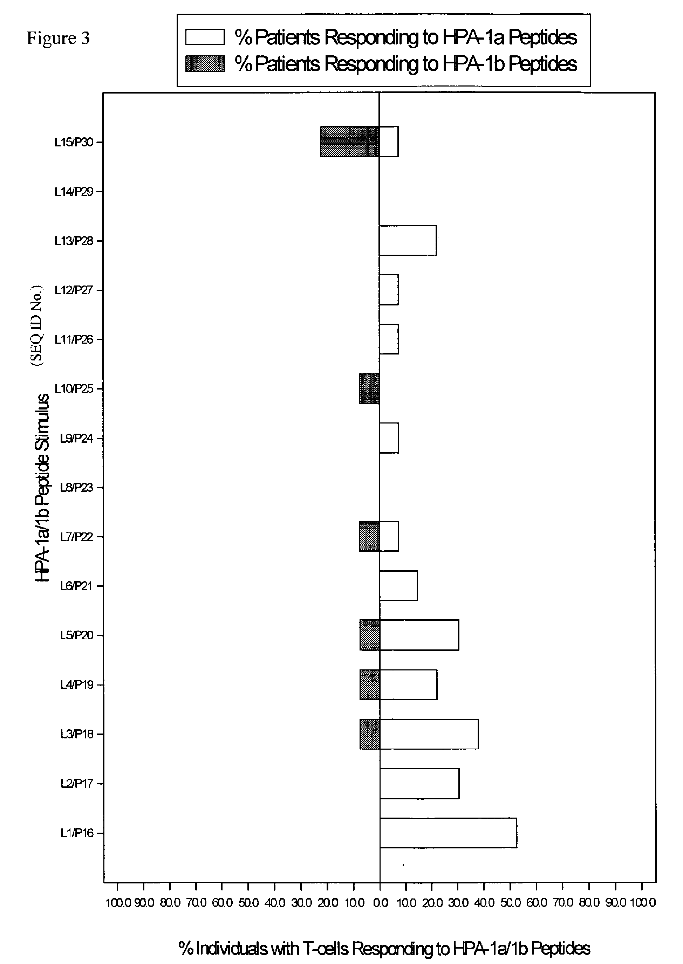

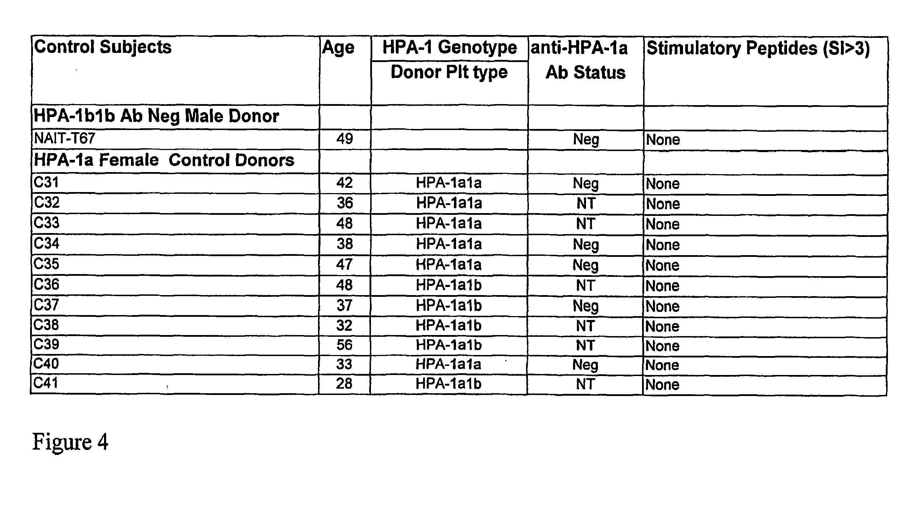

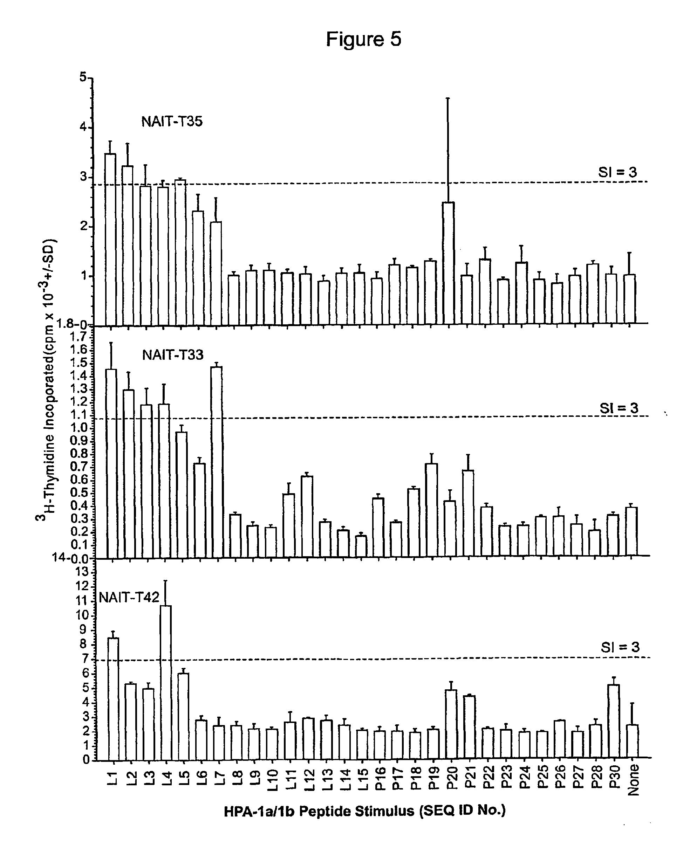

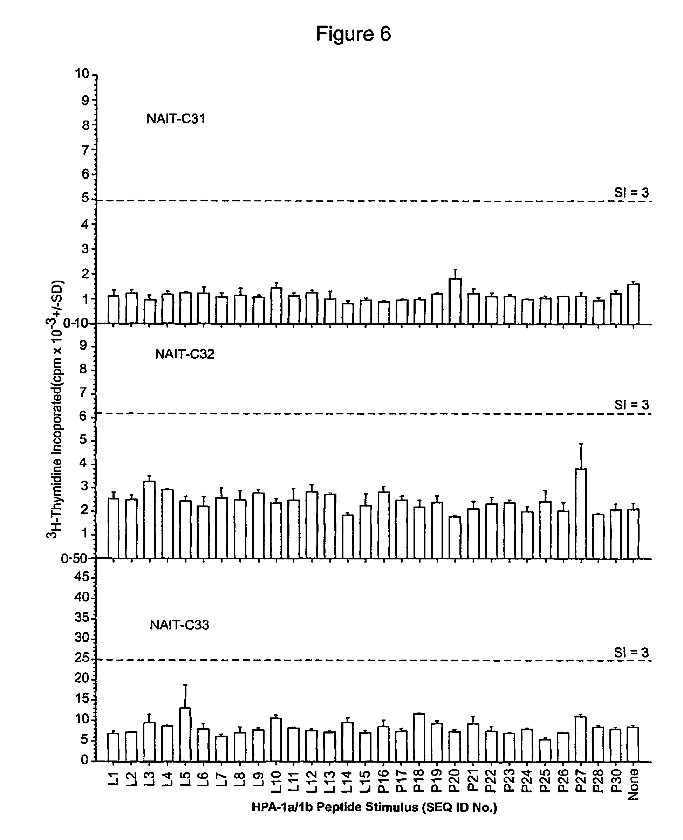

Peripheral blood mononuclear cells from the majority of HPA-1b/1b women who developed anti-HPA-1a alloantibody during pregnancy (9/14) proliferated in response to one or more HPA-1a peptides. Of the 9 responsive women, only 2 responded to HPA-1b peptides in addition to the HPA-1a peptides (see Figure 2). Only a few women (2/7) that did not produce anti-HPA-1a alloantibody during pregnancy responded to both HPA-1a and 1b peptides (see Figures 2 and 3). In contrast, there were no PBMC responses observed in 11 female control donors indicating that the responses were specific (see Figure 4). PBMC from a male HPA 1b1b control did not respond to any of the peptides (results not shown). Representative results from 3 HPA-1a negative women who had produced anti-HPA-1a are shown in Figure 5. Representative results from 3 control donors are shown in Figure 6.

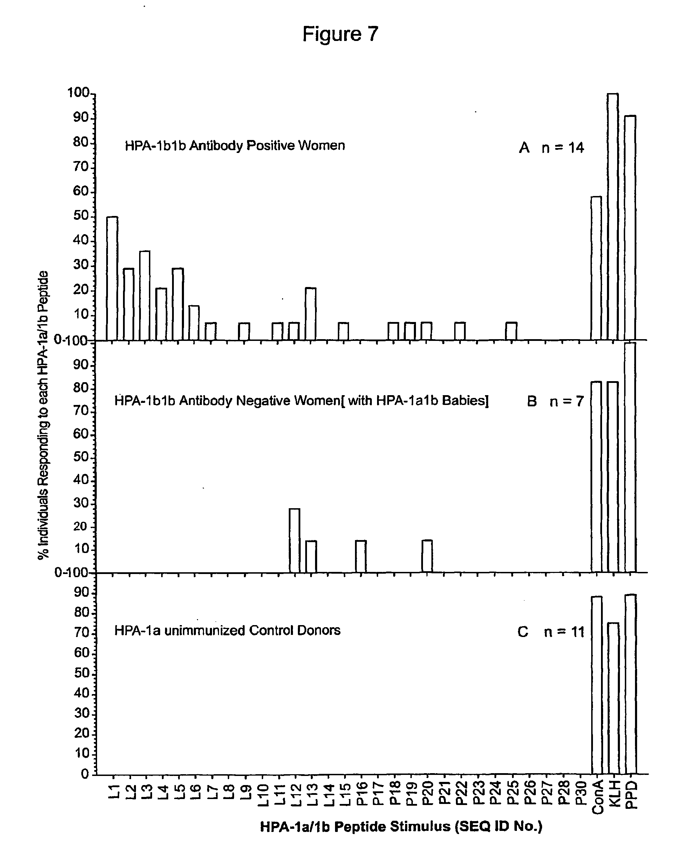

Analysis of T-cell responses from the HPA-1a alloimmunised HPA-1b1b women to HPA-1a peptides revealed that certain peptides induced proliferation more commonly than others. HPA-1a leucine peptides with Leu-33 near the C-terminus were immunodominant. SEQ ID No:1 elicited proliferation in approximately 50% of the HPA-1a alloimmunised HPA-1b1b women. SEQ ID Nos: 1 to 5 stimulated the proliferation of T-cells from 29%, 36%, 21% and 29% of the HPA-1a alloimmunised HPA-1b1b women respectively (Figure 7). SEQ ID Nos 6 and 7, although stimulatory in fewer HPA-1b1b women, did stimulate strong responses in particular individuals, e.g. see patient NAIT-T33 (Figure 5). Other peptides were either unstimulatory or less frequently targeted.

Peripheral blood mononuclear cells from the majority of responsive women exhibited reactivity to HPA-1a peptides rather than to HPA-1b peptides and proliferative responses to HPA-1a peptides were higher compared to those with HPA-1b peptides (Figure 3). Unimmunised controls failed to respond to any of the peptides (Figure 6).

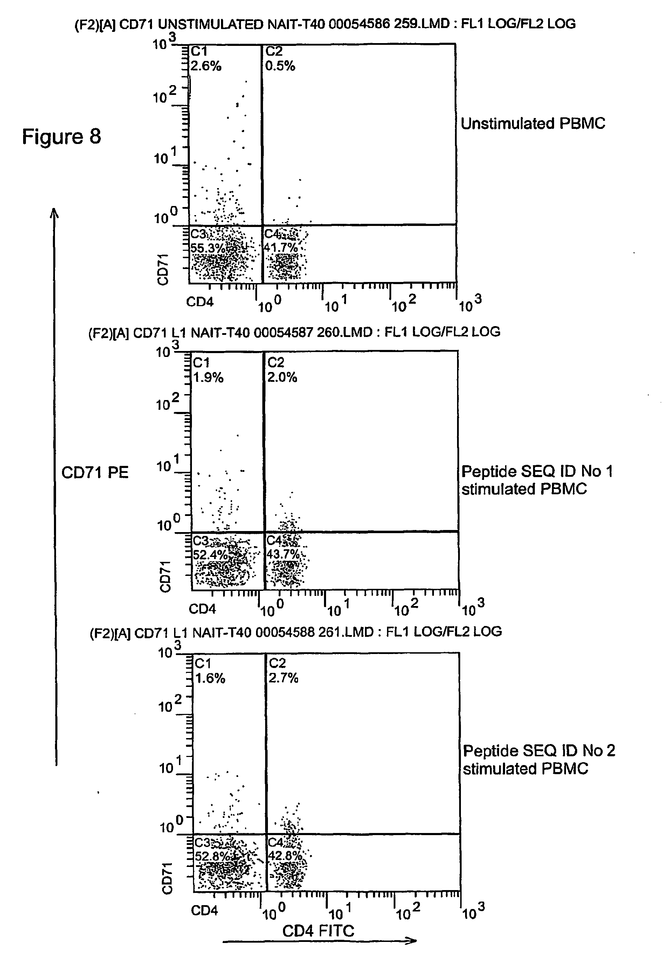

Characterisation of lymphocytes that proliferated in response to HPA-1a peptides by flowcytometry, confirmed that these cells were CD3+, CD4+ T-helper cells which carried the activation marker CD71. Representative results with lymphocytes obtained from an HPA-1a alloimmunised HPA-1b1b woman stimulated with leucine peptides SEQ ID Nos:1 and 2 are shown in Figure 8.

In proliferating cultures, stimulated with peptide, there was an increase in the numbers of CD3+ cells expressing the CD4+ phenotype of helper T-cell and carrying the activation marker CD71. This provides evidence of T-cell involvement in providing B-cell help for the generation of anti-platelet alloantibodies that are associated with FMAIT.

T-cell epitopes from the C-terminal region of the HPA-1a polymorphic region (SEQ ID Nos:1, 2, 3, 4, 5, 6 and 7) are immunodominant in stimulating specific responses in vitro in immunised women, i.e. T-cells recognise peptides containing the polymorphism when the polymorphism is in the C-terminal part of the peptide.

These immunodominant epitopes may be used as a basis for the induction of tolerance to the HPA-1a antigen if administered by a tolerogenic (e.g. mucosal) route, and have potential use in the prevention of FMAIT in susceptible HPA-1a negative women.

These peptides (or their derivatives, or sequences cross-reactive with them) also have potential use for reversal of an established immune response in women who have experienced previous FMAIT, or in patients who have developed resistance to platelet transfusions.

The techniques described here to identify immunodominant epitopes of the HPA-1a antigen can be used to identify similar epitopes of other clinically important HPA antigens on any of the platelet glycoproteins carrying these alleles.

## Claims

1. Use of an immunologically effective human platelet antigen (HPA) or a peptide fragment thereof in the manufacture of a medicament for the prevention or management of a condition caused by exposure to an antithetical allele of a platelet by transfusion or during pregnancy by tolerisation wherein the medicament is formulated for delivery through non-invasive routes.

2. Use according to claim 1 wherein the condition is fetomaternal alloimmune response thrombocytopenia (FMAIT), post-transfusion purpura or platelet refractoriness.

3. Use according to either claim 1 or 2 wherein the HPA is selected from HPA-1a, HPA-1b, HPA-2a, HPA-2b, HPA-3a, HPA-3b, HPA-4a, HPA-4b, HPA-5a, HPA-5b, HPA-6a, HPA-6b, HPA-7a, HPA-7b, HPA-8a, HPA-8b, HPA-9a, HPA-9b, HPA-10a, HPA-10b, HPA-11a, HPA-11b.

4. Use according to any preceding claim wherein the HPA has a genotype HPA-1a.

5. Use according to claim 4 wherein the HPA-1a has sequence SEQ ID No: 1, 2, 3, 4, 5, 6 or 7.

6. Use according to any preceding claim wherein the composition is formulated for delivery through mucosal tissue.

## Patentansprüche

1. Verwendung eines immunologisch effektiven humanen Plättchen-Antigens (HPA) oder eines Peptidfragments dessen in der Herstellung eines Medikaments zur Vorbeugung gegen eine oder zum Management einer Krankheit, die durch Aussetzen an ein antithetisches Allel eines Blutplättchens durch Transfusion oder während der Schwangerschaft durch Erzeugung einer Toleranz, wobei das Medikament zur Verabreichung über nicht-invasive Routen formuliert ist.

2. Verwendung gemäß Anspruch 1, wobei die Krankheit eine fetomaternale Alloimmun-Thormbozytopenie, postransfusionelle Purpura oder Refraktärzustand der Thromobozyten ist.

3. Verwendung gemäß Anspruch 1 oder 2, wobei das HPA aus HPA-1a, HPA-1b, HPA-2a, HPA-2b, HPA-3a, HPA-3b, HPA-4a, HPA-4b, HPA-5a, HPA-5b, HPA-6a, HPA-6b, HPA-7a, HPA-7b, HPA-8a, HPA-8b, HPA-9a, HPA-9b, HPA-10a, HPA-10b, HPA-11a, HPA-11b ausgewählt wird.

4. Verwendung gemäß eines der vorangegangenen Ansprüche, wobei das HPA einen Genotyp HPA-1a hat.

5. Verwendung gemäß Anspruch 4, wobei das HPA-1a die Sequenz SEQ ID Nr.: 1, 2, 3, 4, 5, 6, oder 7 hat.

6. Verwendung gemäß eines der vorangegangenen Ansprüche, wobei die Zusammensetzung zur Verabreichung durch Schleimhautgewebe formuliert ist.

## Revendications

1. Utilisation d'un antigène plaquettaire humain (appelé *human platelet antigen*) (HPA) immunologiquement efficace ou d'un fragment peptidique de celui-ci dans la fabrication d'un médicament pour la prévention ou la prise en charge d'une condition causée par une exposition à un allèle antithétique d'une plaquette par transfusion ou pendant une grossesse par tolérisation dans laquelle le médicament est formulé pour être dispensé par voies d'administration non invasives.

2. Utilisation selon la revendication 1 dans laquelle la condition est une thrombocytopénie par réponse d'alloimmunisation foeto-maternelle (appelée *fetomaternal alloimmune response thrombocytopenia* (FMAIT), un purpura post-transfusionnel ou un état réfractaire plaquettaire.

3. Utilisation selon soit la revendication 1 ou 2 dans laquelle le HPA est choisi à partir de HPA-1a, HPA,1b, HPA-2a, HPA-2b, HPA-3a, HPA-3b, HPA-4a, HPA-4b, HPA-5a, HPA-5b, HPA-Ga, HPA-6b, HPA-7a, HPA-7b, HPA-8a, HPA-8b, HPA-9a, HPA-9b, HPA-10a, HPA-10b, HPA-11a, HPA-11b.

4. Utilisation selon selon l'une des revendications précédentes dans laquelle le HPA a un génotype de HPA-1a.

5. Utilisation selon la revendication 4, dans laquelle le HPA-1a possède une séquence SEQ ID No : 1, 2, 3, 4, 5, 6 ou 7.

6. Utilisation selon l'une quelconque des revendications précédentes dans laquelle la composition est formulée pour être dispensée à travers le tissu muqueux.


### SEQUENCE LISTING

<110> Aberdeen University The Common Services Agency for Scottish Health Service
<120> Pharmaceutical Compositions
<130> P182
<150> GB0315754.2
   <151> 2003-07-04
<160> 30
<170> PatentIn version 3.2
<210> 1
   <211> 15
   <212> PRT
   <213> Homo sapiens
<400> 1
   Val Ser Pro Met Cys Ala Trp Cys Ser Asp Glu Ala Leu Pro Leu
   1               5                   10                  15
<210> 2
   <211> 15
   <212> PRT
   <213> Homo sapiens
<400> 2
   Ser Pro Met Cys Ala Trp Cys Ser Asp Glu Ala Leu Pro Leu Gly
   1 5 10 15
<210> 3
   <211> 15
   <212> PRT
   <213> Homo sapiens
<400> 3
   Pro Met Cys Ala Trp Cys Ser Asp Glu Ala Leu Pro Leu Gly Ser
   1 5 10 15
<210> 4
   <211> 15
   <212> PRT
   <213> Homo sapiens
<400> 4
   Met Cys Ala Trp Cys Ser Asp Glu Ala Leu Pro Leu Gly Ser Pro
   1 5 10 15
<210> 5
   <211> 15
   <212> PRT
   <213> Homo sapiens
<400> 5
   Cys Ala Trp Cys Ser Asp Glu Ala Leu Pro Leu Gly Ser Pro Arg
   1 5 10 15
<210> 6
   <211> 15
   <212> PRT
   <213> Homo sapiens
<400> 6
   Ala Trp Cys Ser Asp Glu Ala Leu Pro Leu Gly Ser Pro Arg Cys
   1 5 10 15
<210> 7
   <211> 15
   <212> PRT
   <213> Homo sapiens
<400> 7
   Trp Cys Ser Asp Glu Ala Leu Pro Leu Gly Ser Pro Arg Cys Asp
   1 5 10 15
<210> 8
   <211> 15
   <212> PRT
   <213> Homo sapiens
<400> 8
   Cys Ser Asp Glu Ala Leu Pro Leu Gly Ser Pro Arg Cys Asp Leu
   1 5 10 15
<210> 9
   <211> 15
   <212> PRT
   <213> Homo sapiens
<400> 9
   Ser Asp Glu Ala Leu Pro Leu Gly Ser Pro Arg Cys Asp Leu Lys
   1 5 10 15
<210> 10
   <211> 15
   <212> PRT
   <213> Homo sapiens
<400> 10
   Asp Glu Ala Leu Pro Leu Gly Ser Pro Arg Cys Asp Leu Lys Glu
   1 5 10 15
<210> 11
   <211> 15
   <212> PRT
   <213> Homo sapiens
<400> 11
   Glu Ala Leu Pro Leu Gly Ser Pro Arg Cys Asp Leu Lys Glu Asn
   1 5 10 15
<210> 12
   <211> 15
   <212> PRT
   <213> Homo sapiens
<400> 12
   Ala Leu Pro Leu Gly Ser Pro Arg Cys Asp Leu Lys Glu Asn Leu
   1 5 10 15
<210> 13
   <211> 15
   <212> PRT
   <213> Homo sapiens
<400> 13
   Leu Pro Leu Gly Ser Pro Arg Cys Asp Leu Lys Glu Asn Leu Leu
   1 5 10 15
<210> 14
   <211> 15
   <212> PRT
   <213> Homo sapiens
<400> 14
   Pro Leu Gly Ser Pro Arg Cys Asp Leu Lys Glu Asn Leu Leu Lys
   1 5 10 15
<210> 15
   <211> 15
   <212> PRT
   <213> Homo sapiens
<400> 15
   Leu Gly Ser Pro Arg Cys Asp Leu Lys Glu Asn Leu Leu Lys Asp
   1 5 10 15
<210> 16
   <211> 15
   <212> PRT
   <213> Homo sapiens
<400> 16
   Val Ser Pro Met Cys Ala Trp Cys Ser Asp Glu Ala Leu Pro Pro
   1 5 10 15
<210> 17
   <211> 15
   <212> PRT
   <213> Homo sapiens
<400> 17
   Ser Pro Met Cys Ala Trp Cys Ser Asp Glu Ala Leu Pro Pro Gly
   1 5 10 15
<210> 18
   <211> 15
   <212> PRT
   <213> Homo sapiens
<400> 18
   Pro Met Cys Ala Trp Cys Ser Asp Glu Ala Leu Pro Pro Gly Ser
   1 5 10 15
<210> 19
   <211> 15
   <212> PRT
   <213> Homo sapiens
<400> 19
   Met Cys Ala Trp Cys Ser Asp Glu Ala Leu Pro Pro Gly Ser Pro
   1 5 10 15
<210> 20
   <211> 15
   <212> PRT
   <213> Homo sapiens
<400> 20
   Cys Ala Trp Cys Ser Asp Glu Ala Leu Pro Pro Gly Ser Pro Arg
   1 5 10 15
<210> 21
   <211> 15
   <212> PRT
   <213> Homo sapiens
<400> 21
   Ala Trp Cys Ser Asp Glu Ala Leu Pro Pro Gly Ser Pro Arg Cys
   1 5 10 15
<210> 22
   <211> 15
   <212> PRT
   <213> Homo sapiens
<400> 22
   Trp Cys Ser Asp Glu Ala Leu Pro Pro Gly Ser Pro Arg Cys Asp
   1 5 10 15
<210> 23
   <211> 15
   <212> PRT
   <213> Homo sapiens
<400> 23
   Cys Ser Asp Glu Ala Leu Pro Pro Gly Ser Pro Arg Cys Asp Leu
   1 5 10 15
<210> 24
   <211> 15
   <212> PRT
   <213> Homo sapiens
<400> 24
   Ser Asp Glu Ala Leu Pro Pro Gly Ser Pro Arg Cys Asp Leu Lys
   1 5 10 15
<210> 25
   <211> 15
   <212> PRT
   <213> Homo sapiens
<400> 25
   Asp Glu Ala Leu Pro Pro Gly Ser Pro Arg Cys Asp Leu Lys Glu
   1 5 10 15
<210> 26
   <211> 15
   <212> PRT
   <213> Homo sapiens
<400> 26
   Glu Ala Leu Pro Pro Gly Ser Pro Arg Cys Asp Leu Lys Glu Asn
   1 5 10 15
<210> 27
   <211> 15
   <212> PRT
   <213> Homo sapiens
<400> 27
   Ala Leu Pro Pro Gly Ser Pro Arg Cys Asp Leu Lys Glu Asn Leu
   1 5 10 15
<210> 28
   <211> 15
   <212> PRT
   <213> Homo sapiens
<400> 28
   Leu Pro Pro Gly Ser Pro Arg Cys Asp Leu Lys Glu Asn Leu Leu
   1 5 10 15
<210> 29
   <211> 15
   <212> PRT
   <213> Homo sapiens
<400> 29
   Pro Pro Gly Ser Pro Arg Cys Asp Leu Lys Glu Asn Leu Leu Lys
   1 5 10 15
<210> 30
   <211> 15
   <212> PRT
   <213> Homo sapiens
<400> 30
   Pro Gly Ser Pro Arg Cys Asp Leu Lys Glu Asn Leu Leu Lys Asp
   1               5                   10                  15